(19) Europäisches Patentamt
European Patent Office
Office européen des brevets

(11) EP 4 575 927 A1

(12) **EUROPEAN PATENT APPLICATION**

(43) Date of publication:
25.06.2025  Bulletin 2025/26

(21) Application number: 24220402.2

(22) Date of filing: 17.12.2024

(51) International Patent Classification (IPC):
*G06N 10/60* (2022.01)

(52) Cooperative Patent Classification (CPC):
**G06N 10/60**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 21.12.2023  IN 202321087619

(71) Applicant: **Tata Consultancy Services Limited Maharashtra (IN)**

(72) Inventors:
• MUKHERJEE, Anirban
400096 Mumbai, Maharashtra (IN)
• GOPAL, Ananthakrishna
400096 Mumbai, Maharashtra (IN)
• BANERJEE, Ritam
400096 Mumbai, Maharashtra (IN)

(74) Representative: **Goddar, Heinz J.**
**Boehmert & Boehmert**
**Anwaltspartnerschaft mbB**
**Pettenkoferstrasse 22**
**80336 München (DE)**

(54) **METHOD AND SYSTEM FOR IMPLEMENTING DENSITY FUNCTIONAL THEORY USING QUANTUM PROCESSORS**

(57)    Conventional methods implement Density Functional Theory (DFT) simulations on classical processors which is time consuming. The disclosure relates to method and system for implementing DFT on quantum processors. Initially, atomic coordinates of each atom of a chemical compound whose properties are to be extracted is received. Electron integrals, a core Hamiltonian, and a collocation matrix are computed from the atomic coordinates. The core Hamiltonian is diagonalized to obtain a density matrix of the chemical compound which is further updated iteratively until a convergence criteria is satisfied. At each iteration, a direct matrix is computed from which correlation exchange matrix is obtained using one or more DFT protocols. Further, the direct and correlation exchange matrix are added to get a Fock matrix which is diagonalized to obtain updated density matrix. Once the convergence criteria is satisfied, a final density matrix is obtained which is used to extract the properties.

FIG. 3

**Description**

CROSS-REFERENCE TO RELATED APPLICATIONS AND PRIORITY

The present application claims priority to Indian application, Application No. 202321087619, filed in India on December 21, 2023.

TECHNICAL FIELD

The disclosure herein generally relates to the field of quantum computing, and, more particularly, to a method and system for implementing Density Functional Theory (DFT) using quantum processors for simulating chemical compounds.

BACKGROUND

Companies in the field of pharma or material sciences have successfully synthesized and predicted several million chemical compounds for practical use. These chemical compounds have been listed across various curated databases. Each molecule in the chemical compound and its associated conformations have several properties such as solubility, stability, binding energy, electron density, atomic spectra, reaction rates, scattering cross-section, response properties and the like. Computing these properties and updating them in the curated databases for future use is performed in the field of classical computational chemistry. All the classical computational chemistry approaches rely on quantum chemistry calculations for describing the electronic density distribution profile which needs to be calculated at different physical conditions. The most practical computations in the pharma and material science field today are carried out by the Kohn Sham (KS)-Density Functional Theory (DFT) and the Hartree-Fock (HF) approach. DFT has achieved a permanent workhorse status to be the primary starting point for simulating molecules and solids.

Most industrially relevant chemical systems in material sciences industry are bulk materials. One supercell made from a collection of neighboring unit cells involves around thousands of atoms which corresponds to several thousands of electronic orbitals even in the least correlated basis. Currently these chemical systems are treated approximately within the Quantum Mechanical (QM) or Molecular Mechanical (MM) approach, where only a subsystem with strong quantum correlations is simulated using DFT and the rest is treated only via the MM technique. Identifying these subsystems requires doing prior MM and/or force field analysis that adds to additional overhead cost. The gap in applying this DFT technology to the complete system arises from the quartic and cubic scaling wall bottleneck i.e. for a 50000 electronic orbital system (corresponding to a supercell of the bulk material), computing even one KS-DFT step would require 0.001 secs on the FUGAKU- the world's second most powerful supercomputer with a Rmax of 442 PETA Flops. For all practical purposes the DFT code should converge within 100 self-consistency steps therefore to simulate one large supercell on FUGAKU would require 0.1 seconds. For real world simulations of chemical compounds, one would require screening across several lakhs of molecular configurations in a supercell and that would require more than one day. The output electronic density computed from DFT needs to be in turn passed into Force-Field or MM modelling suites that then recomputes the geometric positioning of atoms and the DFT energies needs to be recomputed. Hence, for a bit larger system such calculations can enter several days of calculations even on the largest of the supercomputers. Similarly, the chemical systems in Pharma industry that correspond to Protein-drug or Protein-Protein systems involve more than thousands of atoms which corresponds to a several thousands of electronic orbitals. These chemical systems are treated approximately within the QM or MM approach, only a subsystem is treated with strong quantum correlations and is simulated using DFT and the rest is treated only via the MM technique. Even the drug molecules in the pharma industry have more than 500 molecular weight, therefore screening across different drug molecules enters across several days of efforts.

DFT, although a mature technology, has a tremendous bottleneck. If an algorithm can reduce the time computational complexity of DFT for general chemical systems even nominally from cubic to quadratic or linear then several days of calculations can be reduced to a few days or within a day. This will enable faster and more screening in a short duration of time, handling larger QM regions and reduce the product discovery time drastically. In the conventional KS DFT, the computational complexity scales cubically to the system size which is the consequence of the delocalized nature of the wave functions which are the eigen solutions of the Kohn-Sham single particle Hamiltonian. To scale the DFT calculations to large systems, there have been attempts to develop an algorithm which scales linearly with system size. One such technique is ONETEP (Order-N Electronic Total Energy Package). It uses a basis of non-orthogonal generalized Wannier functions (NGWFs) expressed in terms of periodic cardinal sine (psinc) functions, which are in turn equivalent to a basis of plane-waves. ONETEP therefore is a combination of the benefits of linear scaling with a level of accuracy and variational bounds comparable to that of traditional cubic-scaling plane-wave approaches. During the calculation, the density matrix and the NGWFs are optimized with localization constraints. ONETEP optimizes the total energy of the system, ensuring self-consistent convergence of electronic structure. But ONETEP is primarily designed for periodic systems, which can restrict its application to certain materials and structures. Although ONETEP is efficient for large systems, there are still practical limitations to the system size that can be treated, and the method may not be suitable for extremely large systems. Overall, ONETEP is a powerful and efficient method for performing large-scale DFT calculations in condensed matter physics and materials science. Its linear scaling and localized orbital approach make it well-suited for studying complex materials, but its application is primarily limited to periodic systems.

Currently the efforts are towards speeding up the DFT calculations using Exa scale computing: CPU, GPU, MPI, Multiprocessing etc. However, all of these have memory and processing limitations. As quantum hardware and algorithms continue to develop, various industry sectors, particularly the pharmaceutical and material design domains, are applying quantum computation paradigm to their specific problems. There have been attempts made to implement DFT on a combination of classical and quantum processors, for example, US20220012382A1 which implements DFT on a classical processor and optimizes the DFT results on a quantum processor. However, the complex calculations are still performed on a classical processor which doesn't overcome the above mentioned bottlenecks in DFT calculations.

SUMMARY

Embodiments of the present disclosure present technological improvements as solutions to one or more of the above-mentioned technical problems recognized by the inventors in conventional systems. For example, in one embodiment, a method for implementing Density Functional Theory (DFT) using quantum processors for simulating chemical compounds is provided. The method includes receiving, by one or more classical hardware processors, a chemical compound whose one or more properties are to be extracted and obtaining a plurality of atomic coordinates of each of a plurality of atoms comprised in the chemical compound. Further, the method includes determining, by the one or more classical hardware processors, a plurality of electron integrals, a core Hamiltonian, and a collocation matrix from the plurality of atomic coordinates of each of the plurality of atoms comprised in the chemical compound. The collocation matrix comprises a plurality of basis functions of a plurality of atomic orbitals and a plurality of points on a numerical grid. Each of the plurality of basis functions is a Gaussian wave function centered around the plurality of atomic coordinates. Further, the method includes determining, by a plurality of unentangled QPUs, a density matrix of the chemical compound by diagonalizing the core Hamiltonian. Furthermore, the method includes iteratively updating, by the plurality of unentangled QPUs, the density matrix until a convergence criteria is satisfied, to obtain a final density matrix of the chemical compound, by: computing a direct matrix from the density matrix, determining a correlation exchange matrix based on the direct matrix and the collocation matrix using one or more protocols among a plurality of classes of Density Functional Theory (DFT) protocols, computing a Fock matrix by adding the direct matrix and the correlation exchange matrix, and performing a qubitized diagonalization of the Fock matrix to obtain an updated density matrix. The updated density matrix is used in a subsequent iteration, and the updated density matrix obtained upon satisfying the convergence criteria is the final density matrix. Furthermore, the method includes extracting the one or more properties of the chemical compound using the final density matrix.

In another aspect, a system for implementing Density Functional Theory (DFT) using quantum processors for simulating chemical compounds is provided. The system includes one or more classical hardware processors communicably coupled to a plurality of unentangled Quantum Processor Units (QPUs) via one or more communication interfaces, wherein the one or more classical hardware processors comprises at least one memory storing programmed instructions; one or more Input /Output (I/O) interfaces; and one or more hardware processors operatively coupled to the at least one memory, wherein the one or more classical hardware processors and the plurality of unentangled QPUs are configured by the programmed instructions to receive, by the one or more classical hardware processors, a chemical compound whose one or more properties are to be extracted and obtain a plurality of atomic coordinates of each of a plurality of atoms comprised in the chemical compound. Further, the one or more classical hardware processors are configured to determine a plurality of electron integrals, a core Hamiltonian, and a collocation matrix from the plurality of atomic coordinates of each of the plurality of atoms comprised in the chemical compound. The collocation matrix comprises a plurality of basis functions of a plurality of atomic orbitals and a plurality of points on a numerical grid. Each of the plurality of basis functions is a Gaussian wave function centered around the plurality of atomic coordinates. Further, the plurality of unentangled QPUs are configured by the programmed instructions to determine a density matrix of the chemical compound by diagonalizing the core Hamiltonian. Furthermore, the plurality of unentangled QPUs are configured by the programmed instructions to iteratively update the density matrix until a convergence criteria is satisfied, to obtain a final density matrix of the chemical compound, by: computing a direct matrix from the density matrix, determining a correlation exchange matrix based on the direct matrix and the collocation matrix using one or more protocols among a plurality of classes of Density Functional Theory (DFT) protocols, computing a Fock matrix by adding the direct matrix and the correlation exchange matrix, and performing a qubitized diagonalization of the Fock matrix to obtain an updated density matrix. The updated density matrix is used in a subsequent iteration, and the updated density matrix obtained upon satisfying the convergence criteria is the final density matrix. Furthermore, the plurality of unentangled QPUs are configured by the programmed instructions to extract the one or more properties of the chemical compound using the final density matrix.

In yet another aspect, a computer program product including a non-transitory computer-readable medium having embodied therein a computer program for implementing Density Functional Theory (DFT) using quantum processors for simulating chemical compounds is provided. The computer readable program, when executed on a system comprising one or more classical hardware processors communicably coupled to a plurality of unentangled Quantum Processor Units (QPUs) via interfaces, causes the computing device to execute a method for implementing Density Functional Theory (DFT) using quantum processors for simulating chemical compounds. The method comprises receiving, by one or more classical hardware processors, a chemical compound whose one or more properties are to be extracted and obtaining a plurality of atomic coordinates of each of a plurality of atoms comprised in the chemical compound. Further, the method includes determining, by the one or more classical hardware processors, a plurality of electron integrals, a core Hamiltonian, and a collocation matrix from the plurality of atomic coordinates of each of the plurality of atoms comprised in the chemical compound. The collocation matrix comprises a plurality of basis functions of a plurality of atomic orbitals and a plurality of points on a numerical grid. Each of the plurality of basis functions is a Gaussian wave function centered around the plurality of atomic coordinates. Further, the method includes determining, by a plurality of unentangled QPUs, a density matrix of the chemical compound by diagonalizing the core Hamiltonian. Furthermore, the method includes iteratively updating, by the plurality of unentangled QPUs, the density matrix until a convergence criteria is satisfied, to obtain a final density matrix of the chemical compound, by: computing a direct matrix from the density matrix, determining a correlation exchange matrix based on the direct matrix and the collocation matrix using one or more protocols among a plurality of classes of Density Functional Theory (DFT) protocols, computing a Fock matrix by adding the direct matrix and the correlation exchange matrix, and performing a qubitized diagonalization of the Fock matrix to obtain an updated density matrix. The updated density matrix is used in a subsequent iteration, and the updated density matrix obtained upon satisfying the convergence criteria is the final density matrix. Furthermore, the method includes extracting the one or more properties of the chemical compound using the final density matrix.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is a functional block diagram of a system for implementing Density Functional Theory (DFT) using quantum processors for simulating chemical compounds, in accordance with some embodiments of the present disclosure. FIGS. 2A, 2B and 2C, collectively referred to as FIG. 2 is an exemplary flow diagram illustrating a method of implementing Density Functional Theory (DFT) using quantum processors for simulating chemical compounds, in accordance with some embodiments of the present disclosure.
FIG. 3 illustrates an alternative representation of the flow diagram of FIG. 2, in accordance with some embodiments of the present disclosure.
FIG. 4 illustrates a quantum circuit used to compute a direct matrix in the method illustrated in FIG. 2, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The present disclosure provides a method for implementing Density Functional Theory (DFT) using quantum processors for simulating chemical compounds. Kohn-Sham (KS) hybrid Density Functional Theory (DFT) process is initiated from a set of $N_b$ basis functions $$\mathbb{B} = \{\phi_\mu(r)\}_{\mu=1}^{N_b}$$ to compute the core Hamiltonian $h$ which is a $N_b \times N_b$ matrix. $h$ constitutes the free particle energy of electrons and one-body nuclear potential arising from the electron-nucleus interactions, both these terms are independent of electronic density. Therefore h needs to be computed while initiating the self-consistent field (SCF) procedure. The direct ($J$) matrix, exact-exchange (K) matrix are electronic density dependent terms and are computed from the Electronic Repulsion Integral (ERI) tensor. The correlation exchange potential or the correlation exchange matrix ($V^{xc}$) is computed from the electronic density, its gradient and/or the Kinetic energy density.

Together *J*, *K* and $V^{xc}$ make the hybrid Kohn-Sham Fock Matrix functional F as given by equation 1.

$$F[D] = h + J[D] - cK[D] + V^{xc}[D] \qquad \dots\dots (1)$$

In equation 1, D is the one-particle density matrix discretized in the basis of $\{\phi_\mu\}$ and $c \in \mathbb{R}^+$ is the modulation for the exact exchange term. The terms *J*, *K* can be computed from the AO-integral approach using the ERI tensor $\langle ij|kl \rangle$ and D as in equations 2 and 3, respectively.

$$J_{ij} = \sum_{kl} \langle ij|kl \rangle D_{kl} \dots\dots (2)$$

$$K_{ij} = \sum_{kl} \langle ik|jl \rangle D_{kl} \dots (3)$$

In equations 2 and 3, the ERI tensor $\langle ij|kl \rangle$ is obtained by integrating the space of basis functions in $\mathbb{R}^3$ according to equation 4.

$$\langle Ij|kl \rangle = \int d^3\mathbf{r} \, d^3\mathbf{r}' \phi_i(\mathbf{r})\phi_j(\mathbf{r}) \frac{1}{|\mathbf{r}-\mathbf{r}'|} \phi_k(\mathbf{r}')\phi_l(\mathbf{r}') \dots\dots (4)$$

The correlation-exchange potential $V^{xc}$ in the discretized basis $\{\phi_i\}$ is computed by equation 5 where the basis functions are defined according to equation 6.

$$V_{ij}^{xc} = \int_{R^3} d^3\mathbf{r}\,\phi_i(\mathbf{r}) \frac{\delta E^{xc}[\rho(\mathbf{r})]}{\delta\rho(\mathbf{r})} \phi_j(\mathbf{r}) \dots\dots (5)$$

$$\phi_a(\mathbf{r}) = (x - A_x)^{a_x}(y - A_y)^{a_y}(z - A_z)^{a_z} e^{(-\vartheta(x-A_x)^2)} e^{(-\vartheta(z-A_z)^2)} \dots\dots (6)$$

In equation 6, $A_x$, $A_y$, $A_z$ are nuclear coordinate (or atomic coordinates) locations of the Gaussian functions, $a = (a_x, a_y, a_z)$ are the integer cartesian quanta and $\vartheta$ is the cartesian Gaussian exponent. In equation 5, $E^{xc}$ is the exchange energy functional evaluated for the electronic density r and its form depends on the DFT method being used. The ERI tensor is represented using the Cholesky decomposition representation as given by equation 7.

$$\langle Ij|kl \rangle = \sum_P L_{ij}^P L_{kl}^P \dots\dots (7)$$

The Cholesky matrices $L^P$ can be obtained from the Density-Fitting (DF) or the resolution of identity (RI) method as in equation 8.

$$L_{ij}^P = \frac{1}{\sqrt{w_P}} \sum_Q u_{QP} \langle ij|Q \rangle \dots\dots (8)$$

In equation 8, $\langle ij|Q \rangle$ are 3-center 2-electron integrals represented in terms of the auxiliary basis functions $\{\chi_P(r)\}_{P=1}^{Naux}$,

and $u_{QP}$, $w_P$ are obtained from the spectral decomposition of 2-center 2-electron integrals $V_{PQ} = \sum_R u_{PR} w_R u_{QR}^*$ represented in the auxiliary basis. 3-center 2-electron integrals are given by equation 9 and $V_{PQ} = \langle P|Q \rangle$ represents two center two electron integrals given by equation 10.

$$\langle Ij|P \rangle = \int d^3\mathbf{r}\,d^3\mathbf{r}' \frac{\phi_i(\mathbf{r})\phi_j(\mathbf{r})\Lambda_P(\mathbf{r}')}{|\mathbf{r}-\mathbf{r}'|} \dots\dots (9)$$

$$\langle P|Q \rangle = \int d^3 \mathbf{r} \frac{\Lambda_P(\mathbf{r})\Lambda_Q(\mathbf{r})}{|\mathbf{r}-\mathbf{r}'|} \;\ldots\ldots (10)$$

The $J$ and $K$ matrix elements are computed using the RI approach as in equation 11.

$$J_{ij} = \sum_P L_{ij}^P \sum_{kl} L_{kl}^P D_{kl}, \; K_{ij} = \sum_{Pl} L_{il}^P \sum_k L_{jk}^P D_{kl} \;\ldots (11)$$

When the KS-DFT is classically computed, the complexity of computing $J$ and $K$ is $O(pN^2)$ and this complexity resides in computing $K$. Computing h and $V^{xc}$ has lower complexity of $O(N^2)$. Next step of DFT is to solve the generalized eigenvalue problem for the Fock matrix accounting for the overlap between basis functions $S_{ij} = \langle \phi_i|\phi_j \rangle$ and the density matrix $\boldsymbol{D}^l$ of a current iteration as given by equation 12.

$$F\big[\boldsymbol{D}^{(l)}\big]C^{(l+1)} = SC^{(l+1)}\hat{E}^{(l+1)} \;\ldots\ldots (12)$$

In equation 12, the one particle density matrix is $\boldsymbol{D}^{(l)} = C^{(l)}WC^{(l)\dagger}$, where the occupancy matrix $W_{ij} = \theta(\mu - E_i)\delta_{ij}$ fills up $N_e/2$ lowest energy levels below the chemical potential $\mu$, where $N_e$ is number of electrons.

Conventional methods implement the DFT simulations on classical processors such as CPU, GPU etc., which is time consuming. Some state of the art techniques implement DFT on a combination of classical and quantum processors. However, the complex calculations are still performed on a classical processor which doesn't overcome the bottlenecks in DFT calculations. To overcome these drawbacks, the present disclosure provides a method for implementing Density Functional Theory (DFT) using quantum processors for simulating chemical compounds. More specifically, the present disclosure provides a quantum circuit for computing the direct ($J$) matrix efficiently on a quantum processor. Initially, atomic coordinates of each atom in a chemical compound whose one or more properties must be extracted is received. Electron integrals, a core Hamiltonian, and a collocation matrix is computed from the atomic coordinates. The core Hamiltonian is diagonalized to obtain a density matrix of the chemical compound which is further updated iteratively until a convergence criteria is satisfied. At each iteration, a direct matrix is computed from the density matrix, a correlation exchange matrix is computed from the direct matrix and the collocation matrix, a Fock matrix is computed by adding the direct matrix and the correlation exchange matrix and the Fock matrix is diagonalized to obtain updated density matrix which is used in subsequent iteration. This is repeated until norm of a difference between the updated density matrix at a current iteration and the density matrix at a previous iteration is lesser than a predefined threshold to obtain a final density matrix of the chemical compound which can be used to extract one or more properties of the chemical compound.

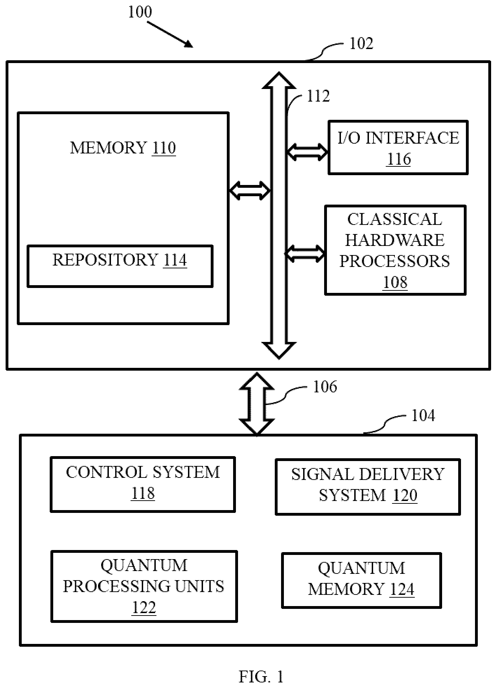

Referring now to the drawings, and more particularly to FIGS. 1 through 4, where similar reference characters denote corresponding features consistently throughout the figures, there are shown preferred embodiments and these embodiments are described in the context of the following exemplary system and/or method.

FIG. 1 is a functional block diagram of a system for implementing Density Functional Theory (DFT) using quantum processors for simulating chemical compounds, in accordance with some embodiments of the present disclosure. The system 100 includes a classical computing system 102, a quantum computing system 104, and a communication interface 106. The classical computing system 102 comprises classical hardware processors 108, at least one memory such as a memory 110, an I/O interface 116. The classical hardware processors 108, the memory 110, and the Input/Output (I/O) interface 116 may be coupled by a system bus such as a system bus 112 or a similar mechanism. In an embodiment, the classical hardware processors 108 can be one or more hardware processors. The terms classical hardware processors and hardware processors are interchangeably used throughout the document. Similarly, the classical computing system is a normal computing system. The I/O interface 116 may include a variety of software and hardware interfaces, for example, a web interface, a graphical user interface, and the like., for example, interfaces for peripheral device(s), such as a keyboard, a mouse, an external memory, a printer, and the like. Further, the I/O interface 116 may enable the system 100 to communicate with other devices, such as web servers, and external databases. The I/O interface 116 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 116 may include one or more ports for connecting several computing systems with one another or to another server computer.

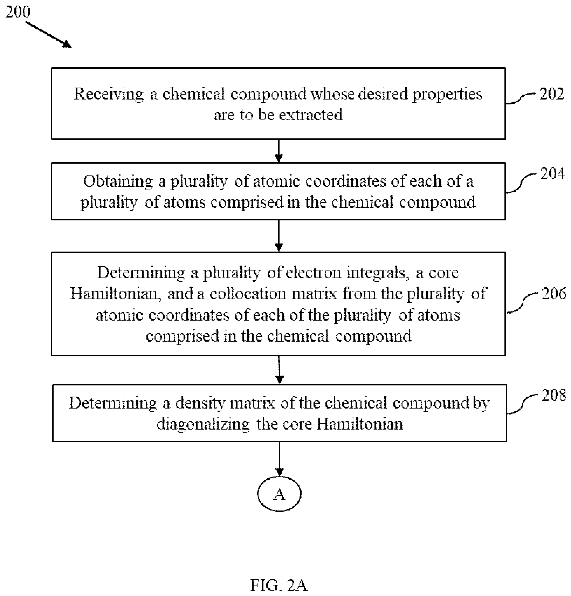

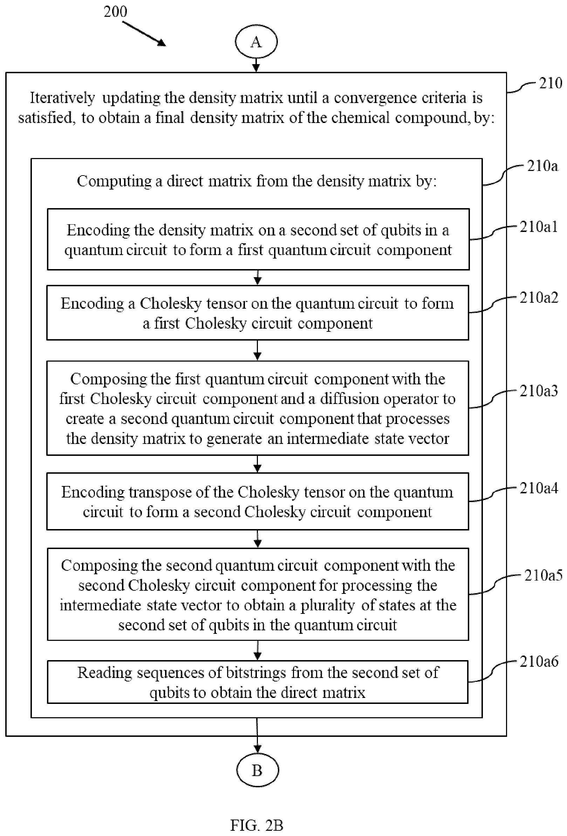

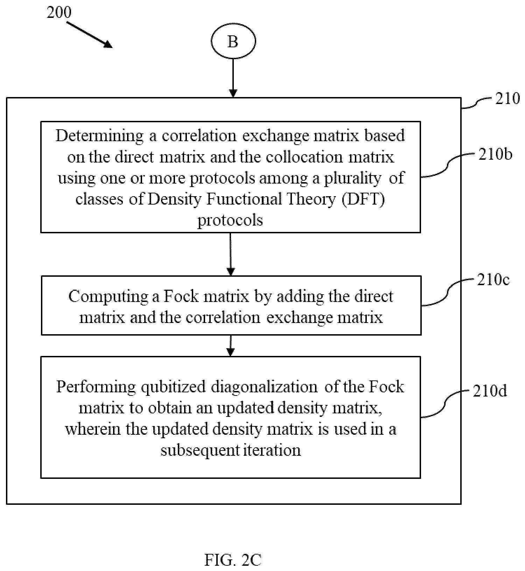

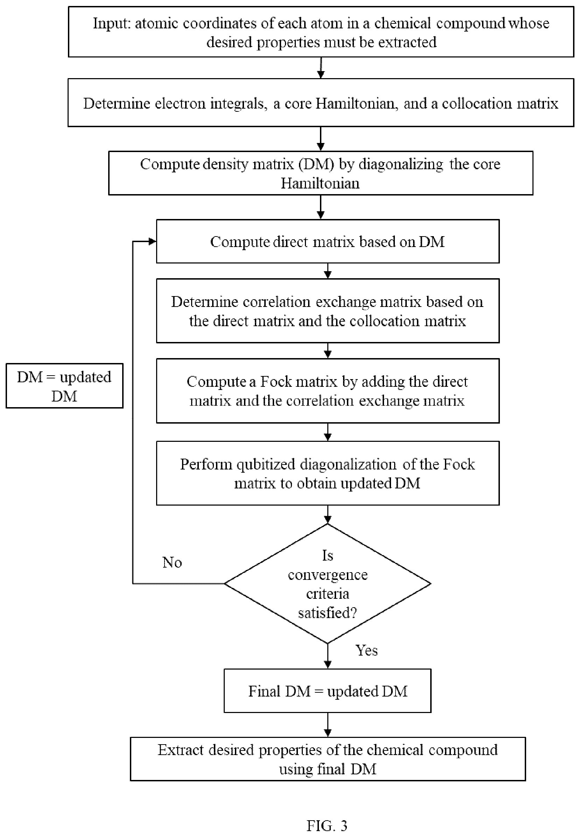

The one or more hardware processors 108 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 108 is configured to fetch and execute computer-readable instructions stored in the memory 110. The memory 110 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 110 includes a data repository 114. The data repository (or repository) 114 may include a plurality of abstracted piece of code for refinement and data that is processed, received, or generated as a result of the execution of the method illustrated in FIGS. 2 and 3. Although the data repository 114 is shown internal to the system 100, it should be noted that, in alternate embodiments, the data repository 114 can also be implemented external to the system 100, where the data repository 114 may be stored within a database (repository 114) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS).

The example quantum computing system 104 shown in FIG. 1 includes a control system 118, a signal delivery system 120, a plurality of Quantum Processing Units (QPUs) 122 and a quantum memory 124. The plurality of QPUs is unentangled and hence alternatively called as the plurality of unentangled QPUs. The quantum computing system 104 may include additional or different features, and the components of a quantum computing system may operate as described with respect to FIG. 1 or in another manner.

The example quantum computing system 104 shown in FIG. 1 can perform quantum computational tasks (such as, for example, quantum simulations or other quantum computational tasks) by executing quantum algorithms. In some implementations, the quantum computing system 104 can perform quantum computation by storing and manipulating information within individual quantum states of a composite quantum system. For example, Qubits (i.e., Quantum bits) can be stored in and represented by an effective two-level sub-manifold of a quantum coherent physical system in the plurality of QPUs 122. In an embodiment, the quantum computing system 104 can operate using gate-based models for quantum computing. For example, the Qubits can be initialized in an initial state, and a quantum logic circuit comprised of a series of quantum logic gates can be applied to transform the qubits and extract measurements representing the output of the quantum computation. The example QPUs 122 shown in FIG. 1 may be implemented, for example, as a superconducting quantum integrated circuit that includes Qubit devices. The Qubit devices may be used to store and process quantum information, for example, by operating as ancilla Qubits, data Qubits or other types of Qubits in a quantum algorithm. Coupler devices in the superconducting quantum integrated circuit may be used to perform quantum logic operations on single qubits or conditional quantum logic operations on multiple qubits. In some instances, the conditional quantum logic can be performed in a manner that allows large-scale entanglement within the QPUs 122. Control signals may be delivered to the superconducting quantum integrated circuit, for example, to manipulate the quantum states of individual Qubits and the joint states of multiple Qubits. In some instances, information can be read from the superconducting quantum integrated circuit by measuring the quantum states of the qubit devices. The QPUs 122 may be implemented using another type of physical system.

The example QPUs 122, and in some cases all or part of the signal delivery system 120, can be maintained in a controlled cryogenic environment. The environment can be provided, for example, by shielding equipment, cryogenic equipment, and other types of environmental control systems. In some examples, the components in the QPUs 122 operate in a cryogenic temperature regime and are subject to very low electromagnetic and thermal noise. For example, magnetic shielding can be used to shield the system components from stray magnetic fields, optical shielding can be used to shield the system components from optical noise, thermal shielding and cryogenic equipment can be used to maintain the system components at controlled temperature, etc.

In the example shown in FIG. 1, the signal delivery system 120 provides communication between the control system 118 and the QPUs 122. For example, the signal delivery system 120 can receive control signals from the control system 118 and deliver the control signals to the QPUs 122. In some instances, the signal delivery system 120 performs preprocessing, signal conditioning, or other operations to the control signals before delivering them to the QPUs 122. In an embodiment, the signal delivery system 120 includes connectors or other hardware elements that transfer signals between the QPUs 122 and the control system 118. For example, the connection hardware can include signal lines, signal processing hardware, filters, feedthrough devices (e.g., light-tight feedthroughs, etc.), and other types of components. In some implementations, the connection hardware can span multiple different temperature and noise regimes. For example, the connection hardware can include a series of temperature stages that decrease between a higher temperature regime (e.g., at the control system 118) and a lower temperature regime (e.g., at the QPUs 122).

In the example quantum computer system 104 shown in FIG. 1, the control system 118 controls operation of the QPUs 122. The example control system 118 may include data processors, signal generators, interface components and other types of systems or subsystems. Components of the example control system 118 may operate in a room temperature regime, an intermediate temperature regime, or both. For example, the control system 118 can be configured to operate at much higher temperatures and be subject to much higher levels of noise than are present in the environment of the QPUs 122. In some embodiments, the control system 118 includes a classical computing system that executes software to compile instructions for the QPUs 122. For example, the control system 118 may decompose a quantum logic circuit or quantum computing program into discrete control operations or sets of control operations that can be executed by the hardware in the QPUs 122. In some examples, the control system 118 applies a quantum logic circuit by generating signals that cause the Qubit devices and other devices in the QPUs 122 to execute operations. For instance, the operations may correspond to single-Qubit gates, two-Qubit gates, Qubit measurements, etc. The control system 118 can generate control signals that are communicated to the QPUs 122 by the signal delivery system 120, and the devices in the QPUs 122 can execute the operations in response to the control signals.

In some other embodiments, the control system 118 includes one or more classical computers or classical computing components that produce a control sequence, for instance, based on a quantum computer program to be executed. For example, a classical processor may convert a quantum computer program to an instruction set for the native gate set or architecture of the QPUs 122. In some cases, the control system 118 includes a microwave signal source (e.g., an arbitrary waveform generator), a bias signal source (e.g., a direct current source) and other components that generate control signals to be delivered to the QPUs 122. The control signals may be generated based on a control sequence provided, for instance, by a classical processor in the control system 118. The example control system 118 may include conversion hardware that digitizes response signals received from the QPUs 122. The digitized response signals may be provided, for example, to a classical processor in the control system 118.

In some embodiments, the quantum computer system 104 includes multiple quantum information processors that operate as respective quantum processor units (QPU). In some cases, each QPU can operate independent of the others. For instance, the quantum computer system 104 may be configured to operate according to a distributed quantum computation model, or the quantum computer system 104 may utilize multiple QPUs in another manner. In some implementations, the quantum computer system 104 includes multiple control systems, and each QPU may be controlled by a dedicated control system. In some implementations, a single control system can control multiple QPUs; for instance, the control system 118 may include multiple domains that each control a respective QPU. In some instances, the quantum computing system 104 uses multiple QPUs to execute multiple unentangled quantum computations (e.g., multiple Variational Quantum Eigen solver (VQE)) that collectively simulate a single quantum mechanical system.

In an embodiment, the quantum memory 124 is a quantum-mechanical version of classical computer memory. The classical computer memory stores information such as binary states and the quantum memory 124 stores a quantum state for later retrieval. These states hold useful computational information known as Qubits. In an embodiment, the communication interface 106 which connects the classical computing system 102 and the quantum computing system 104 is a high speed digital interface.

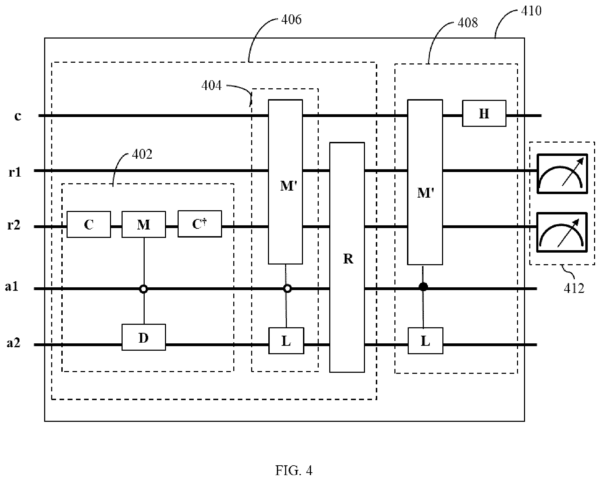

FIGS. 2A, 2B and 2C, collectively referred to as FIG. 2 is an exemplary flow diagram illustrating a method of implementing Density Functional Theory (DFT) using quantum processors for simulating chemical compounds, in accordance with some embodiments of the present disclosure and FIG. 3 is an alternate representation of FIG. 2. In an embodiment, the system 100 includes one or more data storage devices or the memory 110 operatively coupled to the one or more hardware processor(s) 108 and is configured to store instructions for execution of steps of the method 200 by the one or more hardware processors 108. The steps of the method 200 of the present disclosure will now be explained with reference to the components or blocks of the system 100 as depicted in FIG. 1, the steps of flow diagrams as depicted in FIGS. 2 and 3, and the quantum circuit of FIG. 4. The method 200 may be described in the general context of computer executable instructions. Generally, computer executable instructions can include routines, programs, objects, components, data structures, procedures, modules, functions, etc., that perform particular functions or implement particular abstract data types. The method 200 may also be practiced in a distributed computing environment where functions are performed by remote processing devices that are linked through a communication network. The order in which the method 200 is described is not intended to be construed as a limitation, and any number of the described method blocks can be combined in any order to implement the method 200, or an alternative method. Furthermore, the method 200 can be implemented in any suitable hardware, software, firmware, or combination thereof.

Now referring to FIG. 2, at step 202 of the method 200, one or more classical hardware processors are configured to receive a chemical compound whose one or more properties are to be extracted. The chemical compound may be a molecule or a solid, In the context of the present disclosure, the expression 'solid' refers to a periodic arrangement of atoms, for example, a solid may refer to a crystalline form that corresponds to a periodic arrangement of atoms for example. In the pharma industry, the chemical compound may be drug lead and its conformations whose physical properties must be analyzed to determine feasibility of synthesizing a drug from the drug lead. The one or more properties of drug lead includes HOMO-LUMO energies, Dipole moment, partial charges, quasiparticle residue, spin polarization, magnetization. multireference character, etc. Further, at step 204 of the method 200, the one or more classical hardware processors are configured to obtain a plurality of atomic coordinates of each of a plurality of atoms comprised in the chemical compound. In an embodiment, the plurality of atomic coordinates are obtained as an input in the form of a data file or XML file. In another embodiment, tools such as Atomic Simulation Environment (ASE) may be used to obtain the plurality of atomic coordinates.

Further, at step 206 of the method 200, the one or more classical hardware processors are configured to determine a plurality of electron integrals, a core Hamiltonian, and a collocation matrix from the plurality of atomic coordinates of each of the plurality of atoms comprised in the chemical compound. The plurality of electron integrals include a) 4 center 2 electron integrals, b) 3 center 2 electron integrals, c) 2 center 2 electron integrals and d) 2 center 1 electron integrals. The electron integrals a), b), c describe the Coulomb repulsion integral that are computed in atomic orbital basis and can be represented in either spherical or cartesian coordinates. The electron integral d describes the overlap between the basis states. The plurality of electron integrals are determined using tools such as LIBCINT, Python-based Simulations of Chemistry Framework (PySCF), NorthWest computational Chemistry (NWchem) etc. The collocation matrix is a rectangular matrix of dimensions ($Ng$, $Nao$), wherein $Ng$ represents a number of real space grid points, and $Nao$ is a number of basis functions. It comprises a plurality of basis functions of a plurality of atomic orbitals and a plurality of points on numerical grid. Each of the plurality of basis functions is a Gaussian wave function centered around the plurality of atomic coordinates. Further, at step 208 of the method 200, the plurality of unentangled QPUs are configured to determine a density matrix of the chemical compound by diagonalizing the core Hamiltonian. One example way of diagonalizing the core Hamiltonian is described in patent application number 202321061415.

Once the density matrix is determined, at step 210 of the method 200, the plurality of unentangled QPUs are configured to iteratively update the density matrix until a convergence criteria is satisfied to obtain a final density matrix of the chemical compound. The convergence criteria is said to be satisfied when norm of a difference between the updated density matrix at a current iteration and the density matrix at a previous iteration is lesser than a predefined threshold value. Steps 210a to 210d are performed at each iteration to update the density matrix. At step 210a, a direct matrix (alternatively referred to as J matrix, Coulomb matrix etc.) is computed from the density matrix using an example quantum circuit as illustrated in FIG. 4, in an embodiment of the present disclosure. The quantum circuit comprises a first set of qubits, a second set of qubits and a plurality of ancilla qubits. The first set of qubits is associated with number of auxiliary basis functions in density fitting approximation of the plurality electron integrals (more particularly the 3 centered 2 electron integral and 2 centered 2 electron integral), and the second set of qubits is associated with number of basis functions. In FIG. 4, 'c' represents the first set of qubits, 'r1, r2' represent the second set of qubits and 'a1, a2' represent the plurality of ancilla qubits. In an embodiment, the quantum circuit is mathematically represented as: $log\ Naux + 2log\ Nao + 2$ qubits. Initially, at step 210a1, the density matrix is encoded (alternatively referred as loaded or block encoded) on the second set of qubits in the quantum circuit to form a first quantum circuit component (represented by block 402 in FIG. 4). As shown in block 402, the control is loaded on r2 ($C$, $M$ which represents a sequence of multi controlled unitary gates needed to encode the occupancies (1's for occupied electronic states and 0's otherwise), and $C^\dagger$) and data elements of the density matrix are loaded on ancilla qubit a2. The density matrix is loaded using its Eigen decomposition representation that involves: i) $^{Nao}C_2$ Givens rotations on $log\ Nao$ qubits, ii) Then block encoding a diagonal matrix of 1's in the initial $Ne/2$ diagonal bocks and zeros in the rest using $2log\ Nao$ qubits, iii) Another set of $^{Nao}C_2$ Givens rotation with conjugate angles on $Ne/2$ diagonals. This is mathematically represented by equation 13.

$$O(D) = I_2^{\otimes m} \otimes \left[ \left( C \otimes I_2^{\otimes n} \otimes I_2^{a_1} \right) \left( \sum_k |k,k\rangle\langle k,k| \otimes \left( \theta(\mu - E_k)I_{a1} + (1 - \theta(\mu - E_k))Y_{a1} \right) \left( C^\dagger \otimes I_2^{\otimes n} \otimes I_2^{a_1} \right) \right] \otimes I_{a2} \quad \ldots (13)$$

The readouts from the encoding of the density matrix are obtained by equation 14.

$$\langle P, i, 0,0,0 | O(D) | P, j, 0,0,0 \rangle = \sum_{k=1}^{Ne/2} C_{ik} C_{kj}^* = D_{ij}, \text{ where } N_e = 2 \sum_{k=1}^{N} \theta(\mu - E_k)$$
$$\ldots (14)$$

Once the density matrix is encoded, at step 210a2, a Cholesky tensor is encoded on the quantum circuit to form a first Cholesky circuit component (represented by block 404 in FIG. 4). The Cholesky tensor is obtained from one or more electron integrals among the plurality of electron integrals, particularly, 3-center 2-electron and 2-center 2-electron integrals. As shown in the block 404, the controls are encoded on the $log\ Naux + 2\ log\ Nao$ qubits and the data is loaded on the ancilla qubits. Step 210a2 is mathematically represented by equation 15.

$$V_L = \sum_{c=0,r_1=0,r_2=0}^{Naux-1,Nao-1} \left[ |c,r_1,r_2,0\rangle\langle c,r_1,r_2,0| \otimes I_2 + |c,r_1,r_2,1\rangle\langle c,r,1| \otimes \left( L_{r_1,r_2}^{c'} I + i\sqrt{1 - \left(L_{r_1,r_2}^{c'}\right)^2} Y \right) \right] \ldots (15)$$

Further at step 210a3, the first quantum circuit component is composed with the first Cholesky circuit component and a diffusion operator to create a second quantum circuit component that processes the density matrix to generate an intermediate state vector as illustrated by block 406. The diffusion operator R is given by equation 16.

$$R = \sum_{kl} \left[ 2 \frac{|k,+,0\rangle\langle l,+,0|}{Naux} \otimes I_2^{\otimes 2n} - I \right] \ldots\ldots (16)$$

Next, at step 210a4, transpose of the Cholesky tensor is encoded on the quantum circuit to form a second Cholesky circuit component as illustrated by block 408. It is mathematically represented by equation 17.

$$V'_L = \sum_{c=0,r_1=0,r_2=0}^{Naux-1,Nao-1} \left[ |c,r_1,r_2,0\rangle\langle c,r_1,r_2,0| \otimes \left( L_{r_1,r_2}^{c'} I + \right.\right.$$

$$\left.\left. i\sqrt{1 - \left(L_{r_1,r_2}^{c'}\right)^2 Y} \right) + |c,r_1,r_2,1\rangle\langle c,r,1| \otimes I_2 \right] \ldots. (17)$$

Further, at step 210a5, the second quantum circuit component is composed with the second Cholesky circuit component (to form block 410) for processing the intermediate state vector to obtain a plurality of states at the second set of qubits in the quantum circuit. At step 210a6, sequences of bitstrings are read from the second set of qubits (by block 412) to obtain the direct matrix. This step is mathematically represented by equation 18.

$$\langle 0,i,0,0,0| H^{\otimes m} V'_L R V_L O(D) |0,j,0,1,0\rangle = \sum L_{ij}^a L_{kl}^a D_{kl} = J_{ij} \ldots\ldots (18)$$

In equation 18, matrix multiplication and tensor contractions are implemented on the quantum circuit with qubit count efficient resources by applying a theorem which states that if $A$ and $B$ are general rectangular matrices of dimensions $dim(A) = (N, P)$ and $dim(B) = (P, M)$ then there is a unitary operation $U(A, B)$ of dimension $2^{n_q} \times 2^{n_q}$ that operates on a system of $n_q = p + \max(m, n) + 2$ qubit registers $|\cdot\rangle_p|\cdot\rangle_{\max(m,n)}|\cdot\rangle_{a_1}|\cdot\rangle_{a_2}$ (where $n = \lceil log_2 N \rceil, m = \lceil log_2 M \rceil, p = \lceil log_2 P \rceil$) and block encodes the matrix multiplication of $A$ and $B$ such that

$$\left\langle 0|_p \left( \langle i|_{\max(m,n)} \left( \langle 0|_{a_1} \langle 0|_{a_2} U(A,B) |0\rangle_p |j\rangle_{\max(m,n)} \right) |1\rangle_{a_1} \right) |0\rangle_{a_2} \right\rangle = \frac{1}{\max(M,N)P} \frac{|\sum_k A_{ik}B_{kj}}{||A||||B||}.$$

Isometry proof of the theorem is given below-Consider normalized matrices $A' = A/(\sqrt{2}||A||)$, $B' = B/(\sqrt{2}||B||)$ and define corresponding two unitary operators $V(A), V(B)$ according to equations 19 and 20, respectively.

$$V_A = \sum_{c=0,r=0}^{2^p, 2^{\max(m,n)}} [|c,r,0\rangle\langle c,r,0| \otimes \left( A'_{rc} I + i\sqrt{1 - A'^2_{rc}} Y \right) + |c,r,1\rangle\langle c,r,1| \otimes I_2]$$

$$\ldots\ldots (18)$$

$$V_B = \sum_{c=0,r=0}^{2^p, 2^{\max(m,n)}} [|c,r,0\rangle\langle c,r,0| \otimes I_2 + |c,r,1\rangle\langle c,r,1| \otimes \left( B'_{cr} I + i\sqrt{1 - B'^2_{cr}} Y \right)]$$

$$\ldots\ldots (19)$$

Classical data of the B matrix is loaded using the state preparation oracle $V_B H^{\otimes p}$ on the initial state $|0\rangle|j\rangle|1\rangle|0\rangle$ according to equation 20.

$$|\Phi_B\rangle = V_B H^{\otimes p} |0\rangle|j\rangle|1\rangle|0\rangle = \frac{1}{\sqrt{P}} \sum_c [B'_{cj}|c,j,1,0\rangle + \sqrt{1 - B'^2_{cj}}|c,j,1,1\rangle] \ldots\ldots (20)$$

Classical data of the A matrix is loaded using the state preparation oracle $V_A H^{\otimes p}$ according to equation 21.

$$|\Phi_A\rangle = V_A H^{\otimes p}|0\rangle|i\rangle|0\rangle|0\rangle = \frac{1}{\sqrt{P}}\sum_c [A'_{ic}|c, i, 0,0\rangle + \sqrt{1 - A'^2_{ic}}|c, i, 0,1\rangle] \quad \ldots\ldots (21)$$

Note that the states $|\Phi_A\rangle$ and $|\Phi_B\rangle$ are orthogonal, i.e., $\langle\Phi_A|\Phi_B\rangle = 0$. Next, diffusion operator R acting on the row registers and the ancillas $a_1$, $a_2$ is defined by equation 22.

$$R = I_2^{\otimes p} \otimes [H^{\otimes \max(m,n)} \otimes H \otimes I_2)(2|0,0,0\rangle\langle 0,0,0|-1)H^{\otimes \max(m,n)} \otimes H \otimes I_2)] =$$

$$I_2^{\otimes p} \otimes \left[\sum_{k,l} 2\frac{|k,+,0\rangle\langle l,+,0|}{max(M,N)} - I\right] \ldots\ldots (22)$$

Then the overlap between these two states $|\Phi_A\rangle$ and $R|\Phi_B\rangle$ is given by equation 23.

$$\langle\Phi_A|R|\Phi_B\rangle = \langle 0, i, 0,0|H_c^{\otimes p}V_A^\dagger R V_B H_c^{\otimes p}|0, j, 1,0\rangle = \frac{2\sum_c A'_{ic}B'_{cj}}{max(M,N)P} = \frac{\sum_c A_{ic}B_{cj}}{max(M,N)P\|A\|\|B\|}$$

$$\ldots\ldots (23)$$

Hence, by construction it has been proved the existence of $U(A, B)$ can be defined without any isometry according to equation 24.

$$U(A,B) = H_c^{\otimes p}V_A^\dagger R V_B H_c^{\otimes p} \quad \ldots\ldots (24)$$

Once the direct matrix is obtained, at step 210b, a correlation exchange matrix is determined based on the direct matrix and the collocation matrix using one or more protocols among a plurality of classes of Density Functional Theory (DFT) protocols. The plurality of classes of DFT protocols comprise local density approximation (LDA), generalized gradient approximation (GGA), meta GGA, hybrid DFT and double hybrid DFT. If more than one DFT protocols are used for determining the correlation exchange matrix, the correlation exchange matrices obtained from each of the protocols are combined using pre-defined weights. In LDA protocols, the correlation exchange is a function of electronic density. In GGA, the correlation exchange is computed with an additional contribution from the gradient of the electronic density along with the contribution from electronic density. In meta-GGA, an additional contribution from the Hessian of the electronic density is considered along with the contributions from electronic density and its gradient to calculate the exchange correlation. In hybrid DFT, an exact exchange contribution is added in proportion to the direct term for the calculation of energy functional to reduce self-interaction error in LDA, GGA and meta GGA classes. In double Hybrid DFT, an additional wavefunction correction is added to the converged energy obtained from hybrid DFT. This includes post-DFT corrections such as Moller-Plesset Perturbation (MP2).

Once the correlation exchange matrix is determined, at step 210c, a Fock matrix is computed by adding the direct matrix and the correlation exchange matrix. At step 210d, qubitized diagonalization of the Fock matrix is performed to obtain an updated density matrix. The updated density matrix is used in a subsequent iteration. Step 210 is performed iteratively until the convergence criteria is satisfied to get a final density matrix. The one or more properties of the chemical compound are extracted using the final density matrix. Few example properties that can be computed from the density matrix are: 1. HOMO-LUMO gap from the energies of the highest occupied Kohn Sham orbital and lowest unoccupied orbital, 2. The charge distribution from integrating the electronic density in regions around the different atoms where the electronic density in turn is obtained from the density matrix, 3. dipole moment from the expectation value of the perturbation electric field operator with respect to the density matrix.

USE CASE EXAMPLES

Example 1: For drug conformational search and drug design, properties such as molecular descriptors, conformations, solvation energy is obtained from the method 200 disclosed herein. Then from the conformational energies and the descriptors, a subset of drug molecule structure and conformations with desired target properties are prepared via high throughput experimentation.

Example 2: For selecting cathode materials for rechargeable batteries, accurate optimized geometry of the cathode molecules in electrolyte environment, and associated ground state energy are calculated. This will then be used to compute more accurate energy enthalpy differences for the redox reactions. In turn the energy enthalpy differences are used to calculate high Open Cell Voltages (OCV) (equilibrium voltage). A higher OCV leads to a higher cut-off for the recharging voltage. Cathode materials with different co-doped transition metal oxides (like Co, Ni, Mn) are screened using high OCV as the deciding factor. As there are large number of properties to optimize: Number of battery recharging cycles, charging percentage, time to recharge, weight of battery material. The number of battery materials are more than a million, therefore, to screen them faster quantum chemistry calculation based on density functional theory are required which can be efficiently performed by method 200.

EXPERIMENTAL RESULTS

The J matrix computation was performed on classical processor using conventional methods and quantum processor using method 200. The classical complexity is measured in terms of space and time complexity. Similarly, the quantum complexity is measured in terms of number of qubits and gate complexity. The results are recorded in table 1.

Table 1

| Quantity | Classical Complexity | | Quantum Complexity | |
|---|---|---|---|---|
| | Space | Time | No. of Qubits | Gate complexity |
| J(AO) | $O(N_b^4)$ | $O(N_b^4)$ | $4 \log N_b + 2$ | $8 \log N_b$ |
| J(DF) | $O(N_b^2 Naux)$ | $O(N_b^2 Naux)$ | $2 \log N_b + \log Naux$ | $4 \log N_b + 2 \log Naux$ |
| J(sN) | $O(N_b^2 N_g)$ | $O(N_b^2 N_g)$ | $2 \log N_b + \log N_g$ | $4 \log N_b + 2 \log N_g$ |

Table 1 compares the classical and quantum resources needed to compute the J matrix using AO integral approach, Density Fitting (DF) approach and Semi-Numerical (SN) exchange approach. For all these three types of methods to compute J matrix the Quantum circuits of same type as given in FIG. 4 is applicable. For the AO integral approach, the number of qubits needed to encode the electronic integrals is $4 \log N_b + 2$, where $N_b = Nao$ is the number of basis functions. On the other hand, for the classical approach the space complexity is $O(N_b^4)$ to store $N_b^4$ numbers. *log* is clearly a sublinear memory scaling on the other hand the polynomial scaling involved is steep in classical approach. The gate complexity needed for computing the J matrix stems from two sources, one is the diffusion quantum circuit block and the multiqubit gates needed to encode the integrals and the density matrix. The first component from the diffusion quantum circuit block leads to the gate complexity *8log $N_b$*. The second component depends on the molecular system, and the gate complexity for that component depends on the floating-point precision with which we are encoding the integrals associated with the molecule. In comparison the classical time complexity scales as $O(N_b^4)$.

For the Density Fitting (DF) approach, the number of qubits needed to encode the electronic integrals is $2 \log N_b + \log Naux$, where *Naux* is number of auxiliary basis vectors. On the other hand, for the classical approach the space complexity is $O(N_b^2 Naux)$ to store that many numbers. The gate complexity needed for computing the J matrix stems from two sources, one is the diffusion quantum circuit block and the multiqubit gates needed to encode the integrals and the density matrix. The first component from the diffusion quantum circuit block leads to the gate complexity *4log $N_b$ + 2log Naux*. The second component depends on the molecular system, and the gate complexity for that component depends on the floating-point precision with which the integrals associated with the molecule are encoded. In comparison the base classical time complexity scales as $O(N_b^2 Naux)$.

For the Semi Numerical (SN) engine approach, the number of qubits needed to encode the electronic integrals is *2log $N_b$ + log$N_g$*, where $N_g$ number of grid points. On the other hand, for the classical approach the space complexity is $O(N_b^2 N_g)$ to store that many numbers. The gate complexity needed for computing the J matrix stems from two sources, one is the diffusion quantum circuit block and the multiqubit gates needed to encode the integrals and the density matrix. The first component from the diffusion quantum circuit block leads to the gate complexity *2log $N_b$ + log$N_g$*. The second component depends on the molecular system, and the gate complexity for that component depends on the floating-point precision with which we are encoding the integrals associated with the molecule. In comparison the base classical time complexity scales as $O(N_b^2 N_g)$ .

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, non-volatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A quantum simulation method (200), performed by a system comprising one or more classical hardware processors and a plurality of unentangled Quantum Processor Units (QPUs), wherein the one or more classical hardware processors are communicably coupled to the plurality of unentangled QPUs by one or more communication interfaces, wherein the quantum simulation method comprising:

receiving (202), via the one or more classical hardware processors, a chemical compound whose one or more properties are to be extracted;
obtaining (204), via the one or more classical hardware processors, a plurality of atomic coordinates of each of a plurality of atoms comprised in the chemical compound;

determining (206), via the one or more classical hardware processors, a plurality of electron integrals, a core Hamiltonian, and a collocation matrix from the plurality of atomic coordinates of each of the plurality of atoms comprised in the chemical compound, wherein the collocation matrix comprises a plurality of basis functions of a plurality of atomic orbitals and a plurality of points on a numerical grid, wherein each of the plurality of basis functions is a Gaussian wave function centered around the plurality of atomic coordinates;

determining (208), via the plurality of unentangled QPUs, a density matrix of the chemical compound by diagonalizing the core Hamiltonian; and

iteratively updating (210), via the plurality of unentangled QPUs, the density matrix until a convergence criteria is satisfied, to obtain a final density matrix of the chemical compound, by:

i) computing (210a) a direct matrix from the density matrix, by:

encoding (210a1) the density matrix on a second set of qubits in a quantum circuit to form a first quantum circuit component, wherein the quantum circuit comprises a first set of qubits, the second set of qubits, and a plurality of ancilla qubits;

encoding (210a2) a Cholesky tensor on the quantum circuit to form a first Cholesky circuit component, wherein the Cholesky tensor is obtained from one or more electron integrals among the plurality of electron integrals;

composing (210a3) the first quantum circuit component with the first Cholesky circuit component and a diffusion operator to create a second quantum circuit component, wherein the second quantum circuit component processes the density matrix to generate an intermediate state vector;

encoding (210a4) transpose of the Cholesky tensor on the quantum circuit to form a second Cholesky circuit component;

composing (210a5) the second quantum circuit component with the second Cholesky circuit component for processing the intermediate state vector to obtain a plurality of states at the second set of qubits in the quantum circuit; and

reading (210a6) one or more sequences of bitstrings from the second set of qubits to obtain the direct matrix;

ii) determining (210b) a correlation exchange matrix based on the direct matrix and the collocation matrix using one or more protocols among a plurality of classes of Density Functional Theory (DFT) protocols;

iii) computing (210c) a Fock matrix by adding the direct matrix and the correlation exchange matrix; and

iv) performing (210d) a qubitized diagonalization of the Fock matrix to obtain an updated density matrix, wherein the updated density matrix is used in a subsequent iteration, and wherein the updated density matrix obtained upon satisfying the convergence criteria is the final density matrix.

2. The method as claimed in claim 1, comprising extracting, via the one or more classical hardware processors, the one or more properties of the chemical compound using the final density matrix.

3. The method as claimed in claim 1, wherein the collocation matrix is a rectangular matrix of dimensions ($Ng$, $Nao$), and wherein $Ng$ represents a number of real space grid points, and $Nao$ is a number of basis functions.

4. The method as claimed in claim 1, wherein the convergence criteria is satisfied when norm of a difference between the updated density matrix at a current iteration and the density matrix at a previous iteration is lesser than a predefined threshold.

5. The method as claimed in claim 1, wherein the first set of qubits is associated with number of auxiliary basis functions in density fitting approximation of the plurality of electron integrals, and the second set of qubits is associated with number of basis functions.

6. The method as claimed in claim 1, wherein an amplitude amplification is performed on the sequences of bitstrings if there is overlap between the plurality of states.

7. The method as claimed in claim 1, wherein the plurality of classes of DFT protocols comprise local density approximation, generalized gradient approximation, meta generalized gradient approximation, hybrid DFT, and double hybrid DFT, and wherein if more than one DFT protocols are used for determining the correlation exchange matrix, the correlation exchange matrices obtained from each of the protocols are combined using pre-defined weights.

8. A system (100) comprising:
   one or more classical hardware processors (108) and a plurality of unentangled Quantum Processor Units (QPUs) (122), wherein the one or more classical hardware processors (108) are communicably coupled to the plurality of unentangled QPUs (122) by one or more communication interfaces (106), wherein the one or more classical hardware processors are operatively coupled to at least one memory (110) storing programmed instructions and one or more Input/Output (I/O) interfaces (116) and the plurality of unentangled quantum processors (122) are operatively coupled to the at least one quantum memory (124), wherein the one or more hardware processors (108) and the plurality of unentangled QPUs (122) are configured by the programmed instructions to:

   receive a chemical compound whose one or more properties are to be extracted;
   obtain a plurality of atomic coordinates of each of a plurality of atoms comprised in the chemical compound;
   determine a plurality of electron integrals, a core Hamiltonian, and a collocation matrix from the plurality of atomic coordinates of each of the plurality of atoms comprised in the chemical compound, wherein the collocation matrix comprises a plurality of basis functions of a plurality of atomic orbitals and a plurality of points on a numerical grid, wherein each of the plurality of basis functions is a Gaussian wave function centered around the plurality of atomic coordinates;
   determine a density matrix of the chemical compound by diagonalizing the core Hamiltonian; and
   iteratively update the density matrix until a convergence criteria is satisfied, to obtain a final density matrix of the chemical compound, by:

   i) computing (210a) a direct matrix from the density matrix, by:

   encoding (210a1) the density matrix on a second set of qubits in a quantum circuit to form a first quantum circuit component, wherein the quantum circuit comprises a first set of qubits, the second set of qubits, and a plurality of ancilla qubits;
   encoding (210a2) a Cholesky tensor on the quantum circuit to form a first Cholesky circuit component, wherein the Cholesky tensor is obtained from one or more electron integrals among the plurality of electron integrals;
   composing (210a3) the first quantum circuit component with the first Cholesky circuit component and a diffusion operator to create a second quantum circuit component, wherein the second quantum circuit component processes the density matrix to generate an intermediate state vector;
   encoding (210a4) transpose of the Cholesky tensor on the quantum circuit to form a second Cholesky circuit component;
   composing (210a5) the second quantum circuit component with the second Cholesky circuit component for processing the intermediate state vector to obtain a plurality of states at the second set of qubits in the quantum circuit; and
   reading (210a6) one or more sequences of bitstrings from the second set of qubits to obtain the direct matrix;

   ii) determining (210b) a correlation exchange matrix based on the direct matrix and the collocation matrix using one or more protocols among a plurality of classes of Density Functional Theory (DFT) protocols;
   iii) computing (210c) a Fock matrix by adding the direct matrix and the correlation exchange matrix; and
   iv) performing (210d) a qubitized diagonalization of the Fock matrix to obtain an updated density matrix, wherein the updated density matrix is used in a subsequent iteration, and wherein the updated density matrix obtained upon satisfying the convergence criteria is the final density matrix.

9. The system as claimed in claim 8, wherein the one or more hardware processors are configured by the programmed instructions to extract the one or more properties of the chemical compound using the final density matrix.

10. The system as claimed in claim 8, wherein the collocation matrix is a rectangular matrix of dimensions ($Ng$, $Nao$), and wherein $Ng$ represents a number of real space grid points, and $Nao$ is a number of basis functions.

11. The system as claimed in claim 8, wherein the convergence criteria is satisfied when norm of a difference between the updated density matrix at a current iteration and the density matrix at a previous iteration is lesser than a predefined threshold.

12. The system as claimed in claim 8, wherein the first set of qubits is associated with number of auxiliary basis functions in density fitting approximation of the plurality of electron integrals, and the second set of qubits is associated with number of basis functions.

13. The system as claimed in claim 8, wherein an amplitude amplification is performed on the sequences of bitstrings if there is overlap between the plurality of states.

14. The system as claimed in claim 8, wherein the plurality of classes of DFT protocols comprise local density approximation, generalized gradient approximation, meta generalized gradient approximation, hybrid DFT, and double hybrid DFT, and wherein if more than one DFT protocols are used for determining the correlation exchange matrix, the correlation exchange matrices obtained from each of the protocols are combined using pre-defined weights.

15. One or more non-transitory machine-readable information storage mediums comprising one or more instructions which when executed by one or more hardware processors cause:

receiving a chemical compound whose one or more properties are to be extracted;
obtaining a plurality of atomic coordinates of each of a plurality of atoms comprised in the chemical compound;
determining a plurality of electron integrals, a core Hamiltonian, and a collocation matrix from the plurality of atomic coordinates of each of the plurality of atoms comprised in the chemical compound, wherein the collocation matrix comprises a plurality of basis functions of a plurality of atomic orbitals and a plurality of points on a numerical grid, wherein each of the plurality of basis functions is a Gaussian wave function centered around the plurality of atomic coordinates;
determining a density matrix of the chemical compound by diagonalizing the core Hamiltonian; and
iteratively updating the density matrix until a convergence criteria is satisfied, to obtain a final density matrix of the chemical compound, by:

i) computing a direct matrix from the density matrix, by:

encoding the density matrix on a second set of qubits in a quantum circuit to form a first quantum circuit component, wherein the quantum circuit comprises a first set of qubits, the second set of qubits, and a plurality of ancilla qubits;
encoding a Cholesky tensor on the quantum circuit to form a first Cholesky circuit component, wherein the Cholesky tensor is obtained from one or more electron integrals among the plurality of electron integrals;
composing the first quantum circuit component with the first Cholesky circuit component and a diffusion operator to create a second quantum circuit component, wherein the second quantum circuit component processes the density matrix to generate an intermediate state vector;
encoding transpose of the Cholesky tensor on the quantum circuit to form a second Cholesky circuit component;
composing the second quantum circuit component with the second Cholesky circuit component for processing the intermediate state vector to obtain a plurality of states at the second set of qubits in the quantum circuit; and
reading one or more sequences of bitstrings from the second set of qubits to obtain the direct matrix;

ii) determining a correlation exchange matrix based on the direct matrix and the collocation matrix using one or more protocols among a plurality of classes of Density Functional Theory (DFT) protocols;
iii) computing a Fock matrix by adding the direct matrix and the correlation exchange matrix; and
iv) performing a qubitized diagonalization of the Fock matrix to obtain an updated density matrix, wherein the updated density matrix is used in a subsequent iteration, and wherein the updated density matrix obtained upon satisfying the convergence criteria is the final density matrix.

100

102

112

MEMORY 110

REPOSITORY 114

I/O INTERFACE 116

CLASSICAL HARDWARE PROCESSORS 108

106

104

CONTROL SYSTEM 118

SIGNAL DELIVERY SYSTEM 120

QUANTUM PROCESSING UNITS 122

QUANTUM MEMORY 124

FIG. 1

200

| Receiving a chemical compound whose desired properties are to be extracted | 202 |

Obtaining a plurality of atomic coordinates of each of a plurality of atoms comprised in the chemical compound — 204

Determining a plurality of electron integrals, a core Hamiltonian, and a collocation matrix from the plurality of atomic coordinates of each of the plurality of atoms comprised in the chemical compound — 206

Determining a density matrix of the chemical compound by diagonalizing the core Hamiltonian — 208

A

FIG. 2A

200

A

Iteratively updating the density matrix until a convergence criteria is satisfied, to obtain a final density matrix of the chemical compound, by: — 210

Computing a direct matrix from the density matrix by: — 210a

Encoding the density matrix on a second set of qubits in a quantum circuit to form a first quantum circuit component — 210a1

Encoding a Cholesky tensor on the quantum circuit to form a first Cholesky circuit component — 210a2

Composing the first quantum circuit component with the first Cholesky circuit component and a diffusion operator to create a second quantum circuit component that processes the density matrix to generate an intermediate state vector — 210a3

Encoding transpose of the Cholesky tensor on the quantum circuit to form a second Cholesky circuit component — 210a4

Composing the second quantum circuit component with the second Cholesky circuit component for processing the intermediate state vector to obtain a plurality of states at the second set of qubits in the quantum circuit — 210a5

Reading sequences of bitstrings from the second set of qubits to obtain the direct matrix — 210a6

B

FIG. 2B

200

B

Determining a correlation exchange matrix based on the direct matrix and the collocation matrix using one or more protocols among a plurality of classes of Density Functional Theory (DFT) protocols

Computing a Fock matrix by adding the direct matrix and the correlation exchange matrix

Performing qubitized diagonalization of the Fock matrix to obtain an updated density matrix, wherein the updated density matrix is used in a subsequent iteration

210

210b

210c

210d

FIG. 2C

Input: atomic coordinates of each atom in a chemical compound whose desired properties must be extracted

↓

Determine electron integrals, a core Hamiltonian, and a collocation matrix

↓

Compute density matrix (DM) by diagonalizing the core Hamiltonian

↓

Compute direct matrix based on DM

↓

Determine correlation exchange matrix based on the direct matrix and the collocation matrix

↓

Compute a Fock matrix by adding the direct matrix and the correlation exchange matrix

↓

Perform qubitized diagonalization of the Fock matrix to obtain updated DM

↓

Is convergence criteria satisfied?

No → DM = updated DM

Yes ↓

Final DM = updated DM

↓

Extract desired properties of the chemical compound using final DM

FIG. 3

FIG. 4

Europäisches
Patentamt

European
Patent Office

Office européen
des brevets

## EUROPEAN SEARCH REPORT

**Application Number**

EP 24 22 0402

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROSSMANNEK MAX ET AL: "Quantum HF/DFT-embedding algorithms for electronic structure calculations: Scaling up to complex molecular systems", THE JOURNAL OF CHEMICAL PHYSICS, vol. 154, no. 11, 3 September 2020 (2020-09-03), pages 1-16, XP093267641, ISSN: 0021-9606, DOI: 10.1063/5.0029536 * the whole document * | 1-15 | INV. G06N10/60 |
| A | Ko Taehee ET AL: "Implementation of the Density-functional Theory on Quantum Computers with Linear Scaling with respect to the Number of Atoms", arXiv.org, 13 July 2023 (2023-07-13), pages 1-29, XP093268459, Retrieved from the Internet: URL:https://arxiv.org/abs/2307.07067 [retrieved on 2025-04-22] * Abstract, I. Introduction, II. Problem setup, III. Quantum algorithms, IV. Numerical results * | 1-15 | |
| A | ROBERT SCHADE ET AL: "Parallel Quantum Chemistry on Noisy Intermediate-Scale Quantum Computers", ARXIV.ORG, CORNELL UNIVERSITY LIBRARY, 201 OLIN LIBRARY CORNELL UNIVERSITY ITHACA, NY 14853, 11 August 2022 (2022-08-11), XP091292209, * Abstract, II. Reduced density-matrix functional theory, III. Hybrid quantum-classical algorithm for the RDMF * | 1-15 | |

**TECHNICAL FIELDS SEARCHED (IPC)**

G06N

-/--

The present search report has been drawn up for all claims

| Place of search | Date of completion of the search | Examiner |
|---|---|---|
| The Hague | 23 April 2025 | Moro Pérez, Gonzalo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 22 0402

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROSSMANNEK MAX ET AL: "Quantum Embedding Method for the Simulation of Strongly Correlated Systems on Quantum Computers", JOURNAL OF PHYSICAL CHEMISTRY LETTERS, vol. 14, no. 14, 6 February 2023 (2023-02-06), pages 1-8, XP093267643, US ISSN: 1948-7185, DOI: 10.1021/acs.jpclett.3c00330 * page 1 - page 6 * | 1-15 | |
| A | SAMBIT DAS ET AL: "DFT-FE 1.0: A massively parallel hybrid CPU-GPU density functional theory code using finite-element discretization", ARXIV.ORG, CORNELL UNIVERSITY LIBRARY, 201 OLIN LIBRARY CORNELL UNIVERSITY ITHACA, NY 14853, 15 March 2022 (2022-03-15), XP091190622, * Abstract, 2. Real-space Kohn-Sham DFT formulation and finite-element discretization, 3. Numerical methodology, 4. Architecture of the hybrid CPU-GPU implementation * | 1-15 | **TECHNICAL FIELDS SEARCHED (IPC)** |
| A | RYAN PEDERSON ET AL: "Large scale quantum chemistry with Tensor Processing Units", ARXIV.ORG, CORNELL UNIVERSITY LIBRARY, 201 OLIN LIBRARY CORNELL UNIVERSITY ITHACA, NY 14853, 13 December 2022 (2022-12-13), XP091392282, DOI: 10.1021/ACS.JCTC.2C00876 * Abstract, Introduction, Results * | 1-15 | |

The present search report has been drawn up for all claims

| Place of search | Date of completion of the search | Examiner |
|---|---|---|
| The Hague | 23 April 2025 | Moro Pérez, Gonzalo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**EP 4 575 927 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202321087619
- US 20220012382 A1
- WO 202321061415 A